(19) Europäisches Patentamt
European Patent Office
Office européen des brevets

(11) **EP 0 669 164 B1**

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(51) Int. Cl.⁶: **B01J 27/19**, B01J 27/199, C07C 51/25

(21) Anmeldenummer: **95101727.6**

(22) Anmeldetag: **09.02.1995**

(54) **Multimetalloxidmassen**

Multimetal oxide masses

Masses d'oxydes multimétalliques

(84) Benannte Vertragsstaaten:
**BE DE ES FR GB NL**

(30) Priorität: **17.02.1994 DE 4405059**

(43) Veröffentlichungstag der Anmeldung:
**30.08.1995 Patentblatt 1995/35**

(73) Patentinhaber:
**BASF AKTIENGESELLSCHAFT
67056 Ludwigshafen (DE)**

(72) Erfinder:
• **Martin, Dr. Friedrich-Georg
  D-69115 Heidelberg (DE)**
• **Neumann, Dr. Hans-Peter
  D-68307 Mannheim (DE)**
• **Hibst, Dr. Hartmut
  D-69198 Schriesheim (DE)**

(56) Entgegenhaltungen:
EP-A- 0 467 144    EP-A- 0 534 294
EP-A- 0 535 489    EP-A- 0 575 897
FR-A- 2 302 993    US-A- 4 469 810

**Beschreibung**

Die vorliegende Erfindung betrifft Multimetalloxidmassen der allgemeinen Formel I

$$[A]_p [B]_q \qquad\qquad (I),$$

in der die Variablen folgende Bedeutung haben:

| | |
|---|---|
| A | $Mo_{12} X_a^1 X_b^2 X_c^3 X_d^4 S_e X_f^5 O_x$ |
| B | $X_{12}^6 X_g^7 X_h^8 O_y$ |
| $X^1$ | Phosphor, Arsen, Bor, Germanium und/oder Silicium, |
| $X^2$ | Vanadium, Niob und/oder Wolfram, |
| $X^3$ | Wasserstoff, von dem bis zu 97 mol-% ersetzt sein können durch Kalium, Rubidium, Cäsium und/oder Ammonium ($NH_4$), |
| $X^4$ | Antimon und/oder Wismut, |
| $X^5$ | Rhenium und/oder Rhodium, |
| $X^6$ | Molybdän, Wolfram, Niob und/oder Tantal, |
| $X^7$ | Eisen, Kobalt, Nickel, Rhodium, Buthenium, Magnesium, Calcium, Strontium, Barium, Zink, Cadmium, Quecksilber, Yttrium, Scandium und die seltenen Erdmetalle, wobei unter letzteren Cer, Lanthan und Europium bevorzugt werden, |
| $X^8$ | Lithium, Natrium, Kalium, Rubidium, Cäsium und/oder Ammonium ($NH_4$), |
| a | 1 bis 6, vorzugsweise 1 bis 3 und besonders bevorzugt 1,5 bis 2,5, |
| b | 0 bis 6, vorzugsweise 0,2 bis 4 und besonders bevorzugt 0,5 bis 2, |
| c | 3 bis 5, |
| d | 0 bis 6, vorzugsweise 0 bis 3 und besonders bevorzugt 0,5 bis 1,5, |
| e | 0 bis 3, vorzugsweise 0,01 bis 1 und besonders bevorzugt 0,01 bis 0,2, |
| f | 0 bis 3, vorzugsweise 0,01 bis 1 und besonders bevorzugt 0,1 bis 0,5, |
| g | 0,5 bis 20, vorzugsweise 4 bis 15 und besonders bevorzugt 6 bis 12, |
| h | 0 bis 4, vorzugsweise 0,01 bis 3 und besonders bevorzugt 0,01 bis 2, |
| x,y | Zahlen, die durch die Wertigkeit und Häufigkeit der von Sauerstoff verschiedenen Elemente in I bestimmt werden und |
| p,q | von Null verschiedene Zahlen, deren Verhältnis p/q 12:0,1 bis 12:48, vorzugsweise 12:0,25 bis 12:12 und besonders bevorzugt 12:0,5 bis 12:4 beträgt, |

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

$$A\, Mo_{12} X_a^1 X_b^2 X_c^3 X_d^4 S_e X_f^5 O_x$$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

$$B\, X_{12}^6 X_g^7 X_h^8 O_y$$

enthalten, wobei die Bereiche A, B relativ zueinander wie in einem Gemisch aus feinteiligem A und feinteiligem B verteilt sind.

Außerdem betrifft vorliegende Erfindung Verfahren zur Herstellung dieser Massen sowie deren Verwendung.

Die DE-A 26 10 249 und die EP-A 180 997 betreffen Multimetalloxidmassen, deren Element-Bruttozusammensetzung derjenigen der erfindungsgemäßen Multimetalloxidmassen entspricht.

Die Herstellung dieser Multimetalloxidmassen erfolgt dadurch, daß man geeignete Quellen der Bestandteile der gewünschten Multimetalloxidmassen in den erforderlichen Mengen zu einem innigen Trockengemisch verarbeitet und dieses anschließend bei erhöhter Temperatur mehrere Stunden calciniert. Die resultierenden Multimetalloxidmassen werden u.a. als Katalysatoren zur gasphasenkatalytisch oxidativen Herstellung von Methacrylsäure aus Methacrolein empfohlen. Nachteilig an den Multimetalloxidmassen des Standes der Technik ist jedoch, daß sowohl ihre Aktivität als auch die Selektivität der Methacrylsäurebildung bei vorgegebenem Umsatz nicht voll zu befriedigen vermag. Desgleichen gilt für die Reproduzierbarkeit ihrer Herstellung sowie für die Standzeiten während ihres Gebrauchs. Die Standzeiten vermögen insbesondere dann nicht zu befriedigen, wenn die Methacrolein als Hauptbestandteil umfassenden Reaktionsgase als Nebenbestandteile organische Säuren beinhalten.

Aufgabe der vorliegenden Erfindung war daher, Multimetalloxidmassen zur Verfügung zu stellen, die die Nachteile der Multimetalloxidmassen des Standes der Technik nicht aufweisen.

Demgemäß wurden die eingangs definierten Massen I gefunden. Ähnliche Multimetalloxidmassen mit mehrphasigem Aufbau beschreiben die EP-A 668 103 und die EP-A 668 102. Die EP-A 835, die DE-C 3 338 380, die DE-A 42 20 859 und die ältere Anmeldung DE-A 43 07 381 betreffen ebenfalls als Katalysatoren für die gasphasenkatalytisch oxidative Herstellung von Methacrylsäure aus Methacrolein geeignete Multimetalloxidmassen, die in vorteilhafter Weise gleichfalls einen zweiphasigen Aufbau aufweisen, doch ist die Elementzusammensetzung der Phasen dieser Multimetalloxidmassen des Standes der Technik von derjenigen der erfindungsgemäßen Multimetalloxidmassen I verschieden.

Vorteilhafte Massen I sind solche, in denen $X^1$ Phosphor ist. Ferner sind solche Massen I günstig, in denen $X^2$ Vanadium ist. Auch ist es von Vorteil, wenn 3 bis 30 mol-% an $X^3$ Kalium, Rubidium, Cäsium und/oder Ammonium sind. Als bevorzugter Wasserstoffersatz wird Cäsium verwendet. $X^4$ ist vorzugsweise Antimon und $X^5$ ist mit Vorteil Rhodium. Als $X^6$ kommt in vorteilhafter Weise Molybdän zum Einsatz und als $X^7$ werden vorzugsweise Eisen, Kobalt und/oder Nickel angewendet, während es sich bei $X^8$ mit Vorteil um Kalium und/oder Cäsium handelt.

Ferner ist es von Vorteil, wenn wenigstens einer der beiden Anteile $[A]_p$, $[B]_q$ der erfindungsgemäßen Multimetalloxidmassen in den letzteren in Form dreidimensional ausgedehnter Bereiche der chemischen Zusammensetzung A bzw. B enthalten ist, deren Größtdurchmesser $d_A$ bzw. $d_B$ (längste durch den Schwerpunkt des Bereichs gehende Verbindungsstecke zweier auf der Oberfläche (Grenzfläche) des Bereichs befindlicher Punkte) > 0 bis 200 $\mu$m, vorzugsweise 1 bis 200 $\mu$m beträgt. Ein besonders günstiger Größtdurchmesserbereich ist 1 bis 50 $\mu$m und ganz besonders vorteilhaft ist der Bereich 1 bis 30 $\mu$m. Selbstverständlich können die Größtdurchmesser aber auch 50 bis 150 $\mu$m oder 75 bis 125 $\mu$m betragen (die experimentelle Ermittlung der Größtdurchmesser gestattet z.B. die Methode der energiedispersen Röntgenanalyse (EXDS), z.B. an einer Elektronenstrahl-Mikrosonde JEOL JCXA/733).

Vorzugsweise liegt sowohl der Anteil $[A]_p$ als auch der Anteil $[B]_q$ in den erfindungsgemäßen Multimetalloxidmassen im wesentlichen in kristalliner Form vor. D.h. in der Regel bestehen sowohl die Bereiche A als auch die Bereiche B im wesentlichen aus kleinen Kristalliten, deren Größtausdehnung in typischer Weise 0,1 bis 1 $\mu$m beträgt.

Von besonders günstiger Beschaffenheit sind solche Multimetalloxidmassen, deren Bereiche A im wesentlichen aus Kristalliten bestehen, deren Strukturtyp demjenigen des Ammoniumsalzes der Molybdatophosphorsäure $((NH_4)_3PO_4(MoO_3)_{12} \cdot 4H_2O)$ entspricht. Das Vorhandensein dieses Kristallstrukturtyps läßt sich z.B. dadurch nachweisen, daß die Röntgenbeugungsaufnahme der erfindungsgemäßen Multimetalloxidmasse das Beugungsmuster des Ammoniumsalzes der Molybdatophosphorsäure (Fingerabdruck) enthält, wobei je nach Elementzusammensetzung geringe Unterschiede bezüglich der Intensität und der Lage der Beugungslinien möglich sind. Der Röntgenbeugungsfingeraodruck des Ammoniumsalzes der Molybdatophosphorsäure ist z.B. veröffentlicht als Karteikarte 9-412 der JCPDS-ICDD Kartei (1991), die dem Fachmann bekannt und allgemein zugänglich ist. Eine andere Quelle ist Natl. Bur. Stand. (U.S.), Circ. 539, 8 10 (1959). Umfaßt der Anteil $[A]_p$ das Element Antimon, wird dieses im Unterschied zu den übrigen möglichen Konstituenten dieses Anteils nicht in die den Strukturtyp des Ammoniumsalzes der Molybdatophosphorsäure aufweisenden Kristallite eingebaut und befindet sich an der Oberfläche dieser Kristallite bzw. in deren Zwischenräumen. Von Vorteil ist es, wenn sich das Antimon zu 85 bis 95 % seines Gewichtes als Senarmontit (eine kristalline Erscheinungsform des Antimontrioxids) in Räumen zwischen aus im wesentlichen den übrigen Elementen gebildeten Kristalliten des Strukturtyps des Ammoniumsalzes der Molybdatophosphorsäure befindet, während 5 bis 15 % seines Gewichtes in der Oberfläche solcher Kristallite in amorpher Lösung vorliegen (die Herstellung von Senarmontit enthaltenden Multimetalloxidmassen lehrt die ältere Anmeldung DE-A 43 29 907 (O.Z. 0050/44276).

Ferner sind solche erfindungsgemäßen Multimetalloxidmassen bevorzugt, deren Bereiche B im wesentlichen aus Kristalliten bestehen, bei denen ein dem Scheelit verwandter Strukturtyp (Defekt-Scheelit, entweder statistisch oder mit Überstruktur; z.B. vom $Fe_2(MoO_4)_3$-Typ (Karteikarten 31-642 und 35-183 der JCPDS-ICDD Kartei (1991)), vom $Bi_2(MoO_4)_3$-Typ oder vom $Eu_2(WO_4)_3$-Typ) vorliegt. Neben den Defekt-Scheeliten können noch Beimengungen mit Scheelit-($CaWO_4$, Karteikarte 41-1431 der JCPDS-ICDD Kartei (1991)), Wolframit-($MgWO_4$), verzerrtem Wolframit - ($CoMoO_4$) oder $MnMoO_4$-Typ auftreten. Insbesondere sind hier die Hoch- und Tieftemperaturmodifikationen von $\alpha$-$FeMoO_4$ (Karteikarte 22-1115 der JCPDS-ICDD Kartei (1989), $\beta$-$FeMoO_4$ (Karteikarte 22-628 der JCPDS-ICDD Kartei (1991)), $\alpha$-$CoMoO_4$ und $\beta$-$CoMoO_4$ geeignet.

Die erfindungsgemäßen Massen I sind in einfacher Weise z.B. dadurch erhältlich, daß man die Oxometallate

$$X^6_{12}\ X^7_g\ X^8_h\ O_y \qquad\qquad (B)$$

zunächst in feinteiliger Form getrennt vorbildet (Ausgangsmasse 1).

Die Oxometallate B können dadurch hergestellt werden, daß man von geeigneten Quellen ihrer elementaren Konstituenten ein möglichst inniges, vorzugsweise feinteiliges, Trockengemisch herstellt und dieses bei Temperaturen von 350 bis 500°C mehrere Stunden calciniert (prinzipiell kann auch ein nicht calciniertes durch Sprühtrocknung einer wäßrigen Lösung oder Suspension erhaltenes Trockengemisch als Ausgangsmasse 1 eingesetzt werden, die calcinierte Variante ist jedoch bevorzugt). Die Calcinierung kann unter Inertgas, aber auch unter einem Gemisch aus Inertgas und Sauerstoff wie z.B. Luft erfolgen. In der Regel nimmt die erforderliche Calcinationsdauer mit zunehmender Calcinierungstemperatur ab. Wesentlich ist, daß es sich bei den Elementquellen entweder bereits um Oxide handelt, oder um solche Verbindungen, die durch Erhitzen, gegebenenfalls in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht. Besonders geeignete Ausgangsverbindungen der nachfolgenden Elemente sind:

| Mo: | Ammoniumheptamolybdat, |
|---|---|
| Fe: | Eisennitrat (wäßrige Lösung), |
| Co: | Kobaltnitrat (wäßrige Lösung), |
| Alkalimetall: | Alkalimetallnitrat, |
| Rh: | Rhodiumchlorid, |
| Re: | Rheniumpentoxid oder Ammoniumperrhenat. |

Das innige Vermischen der Ausgangsverbindungen kann in trockener oder in nasser Form erfolgen. Erfolgt es in trockener Form, werden die Ausgangsverbindungen zweckmäßigerweise als feinteilige Pulver eingesetzt. Vorzugsweise erfolgt das innige Vermischen jedoch in nasser Form. Üblicherweise werden die Ausgangsverbindungen dabei in Form einer wäßrigen Lösung und/oder Suspension miteinander vermischt. Nach Abschluß des Mischvorgangs wird die fluide Masse getrocknet und nach Trocknung calciniert. Vorzugsweise erfolgt die Trocknung durch Sprühtrocknung (Eingangstemperatur: 250 bis 600°C, Ausgangstemperatur: 80 bis 130°C). Nach erfolgter Calcinierung kann nochmals zerkleinert (z.B. durch Naß- oder Trockenmahlen, z.B. in der Kugelmühle oder durch Strahlmahlen) und aus dem dabei erhältlichen, in der Regel aus im wesentlichen kugelförmigen Partikeln bestehenden, Pulver die Kornklasse mit einem im für die Masse I gewünschten Größtdurchmesserbereich liegenden Korngrößtdurchmesser (in der Regel > 0 bis 200 $\mu$m, üblicherweise 1 bis 200 $\mu$m, vorzugsweise 1 bis 50 $\mu$m und besonders bevorzugt 1 bis 30 $\mu$m) durch in an sich bekannter Weise durchzuführendes Klassieren (z.B. Naß- oder Trockensiebung) abgetrennt werden. Die einzelne Pulverpartikel besteht in der Regel aus zahlreichen Kristalliten, deren Größtausdehnung in typischer Weise 0,1 bis 1 $\mu$m beträgt.

In entsprechender Weise wird aus in gleicher Weise geeigneten Quellen der elementaren Konstituenten der Oxometallate A

$$Mo_{12} \, X_a^1 \, X_b^2 \, X_c^3 \, X_d^4 \, Se \, X_f^5 \, Ox \tag{A}$$

ein feinteiliges inniges Trockengemisch erzeugt, das in der Regel jedoch nicht vorcalciniert wird (Ausgangsmasse 2). Wird die Ausgangsmasse 2 vorcalciniert, so erfolgt die Calcinierung zweckmäßig bei 250 bis 450°C (z.B. unter Inertgas, Luft). Besonders geeignete Ausgangsverbindungen sind:

| Mo: | Ammoniumheptamolybdat, |
|---|---|
| V : | Ammoniummetavanadat, |
| P : | 70 bis 100 gew.-%ige, vorzugsweise 76 bis 85 gew.-%ige Phosphorsäure, |
| Sb: | Senarmontit, |
| S : | Ammoniumsulfat, |
| Re: | Rheniumpentoxid oder Ammoniumperrhenat, |
| B: | Borsäure, |
| As: | Arsentrioxid, |
| Si: | Wasserglas, |
| Nb: | Ammoniumnioboxalat oder Ammoniumniobat, |
| Alkalimetalle: | Alkalimetallnitrate, |
| NH$_4$: | Ammoniumsulfat, -nitrat oder -carbonat, |
| Bi: | Wismutnitrat |

Die Ausgangsmasse 1 und die Ausgangsmasse 2 werden anschließend im gewünschten Mengenverhältnis naß oder trocken miteinander vermischt (vorzugsweise trocken), das Gemisch geformt und dann bei Temperaturen von 250 bis 450°C mehrere Stunden calciniert. Die Calcinierung kann unter Inertgas, aber auch unter einem Gemisch aus Inertgas und Sauerstoff wie z.B. Luft erfolgen.

Das Formen des Gemisches aus der Ausgangsmasse 1 und der Ausgangsmasse 2 kann z.B. durch verdichten (z.B. Tablettieren, Extrudieren oder Strangpressen), gegebenenfalls unter Zusatz an sich üblicher Hilfsmittel, z.B. Graphit oder Stearinsäure als Gleitmittel, erfolgen. Im Falle von Vollkatalysatoren erfolgt das Verdichten unmittelbar zur gewünschten Katalysatorgeometrie, wobei als solche Hohlzylinder mit einem Außendurchmesser und einer Länge von 2 bis 10 mm und einer Wandstärke von 1 bis 3 mm bevorzugt werden. Ganz generell kann das Gemisch aus der Ausgangsmasse 1 und der Ausgangsmasse 2 sowohl vor als auch nach dem Calcinieren geformt werden. Dies kann z.B. auch so erfolgen, daß man das Gemisch nach dem Calcinieren zerkleinert und auf inerte Träger zur Herstellung von Schalenkatalysatoren aufbringt. Das Aufbringen kann aber auch bereits vor der abschließenden Calcinierung erfolgen. In diesem Fall erfolgt das Aufbringen vorzugsweise gemäß der EP-B 293 859. Selbstverständlich können die erfindungsgemäßen Massen I auch in Pulverform eingesetzt werden.

Die erfindungsgemäßen Massen I eignen sich insbesondere als Katalysatoren mit erhöhter Selektivität bei vorgegebenem Umsatz, erhöhter Aktivität, verlängerter Standzeit und verbesserter Reproduzierbarkeit in der Herstellung zur gasphasenkatalytisch oxidativen Herstellung von Methacrylsäure aus Methacrolein.

Die katalytische Gasphasenoxidation von Methacrolein zu Methacrylsäure unter Anwendung der erfindungsgemäßen Katalysatoren kann in an sich bekannter, z.B. in der DE-A 40 22 212 dargestellter, Weise erfolgen.

Desgleichen gilt für die Abtrennung der Methacrylsäure aus dem Produktgasstrom. Das Oxidationsmittel Sauerstoff kann z.B. in Form von Luft, aber auch in reiner Form eingesetzt werden.

Aufgrund der hohen Reaktionswärme werden die Reaktionspartner vorzugsweise mit Inertgas wie $N_2$, $CO_2$, gesättigten Kohlenwasserstoffen und/oder mit Wasserdampf verdünnt.

Vorzugsweise wird bei einem Methacrolein : Sauerstoff : Wasserdampf : Inertgas Verhältnis von 1:(1 bis 3) : (2 bis 20) : (3 bis 30), besonders bevorzugt von 1:(1 bis 3) : (3 bis 10) : (7 bis 18) gearbeitet. Das eingesetzte Methacrolein kann auf verschiedene Weise erhalten worden sein, z.B. durch Gasphasenoxidation von Isobuten, tert.-Butanol oder dem Methylether von tert.-Butanol. Mit Vorteil wird Methacrolein eingesetzt, das durch Kondensation von Propanol mit Formaldehyd in Gegenwart von sekundären Aminen und Säuren in flüssiger Phase gemäß den in der DE-PS 875 114 oder in der DE-AS 28 55 514 beschriebenen Verfahren erhältlich ist. Die Gasphasenoxidation kann sowohl in Wirbelschichtreaktoren als auch in Festbettreaktoren ausgeführt werden. Vorzugsweise wird sie in Rohrbündelreaktoren durchgeführt, in deren Röhren die Katalysatormasse, vorzugsweise in Gestalt zylindrisch geformter Partikel, fest angeordnet ist. Die Reaktionstemperatur beträgt in der Regel 250 bis 350°C, der Reaktionsdruck liegt üblicherweise im Bereich von 1 bis 3 bar und die Gesamtraumbelastung beträgt vorzugsweise 800 bis 1800 Nl/l/h. Unter diesen Bedingungen liegt der Methacroleinumsatz bei einfachem Reaktordurchgang üblicherweise bei 60 bis 90 mol-%. Interessanterweise behalten die erfindungsgemäßen Massen ihre Eigenschaften über eine erhöhte Betriebsdauer im wesentlichen unverändert bei.

Bei dem beschriebenen Verfahren wird üblicherweise jedoch nicht reine Methacrylsäure, sondern ein Produktgemisch erhalten, von welchem die Methacrylsäure nachfolgend abgetrennt werden muß. Dies kann in an sich bekannter Weise erfolgen, z.B. indem man die Reaktionsgase nach indirekter und/oder direkter Kühlung bei Temperaturen von 40 bis 80°C mit Wasser wäscht, wobei eine wäßrige Methacrylsäurelösung erhalten wird, aus der die Methacrylsäure üblicherweise durch Extraktion mit einem organischem Lösungsmittel entfernt und von selbigem durch Destillation abgetrennt wird.

Neben der gasphasenkatalytischen Oxidation von Methacrolein zu Methacrylsäure vermögen die erfindungsgemäßen Massen I aber auch die gasphasenkatalytische Oxidation- und Ammonoxidation von anderen gesättigten, ungesättigten und aromatischen Kohlenwasserstoffen, Alkoholen, Aldehyden und Aminen zu katalysieren.

Genannt sei insbesondere die gasphasenkatalytische Oxidation von anderen 3 bis 6 C-Atome aufweisenden Alkanen, Alkanolen, Alkanalen, Alkenen und Alkenolen (z.B. Propylen, Acrolein, tert.-Butanol, Methylether des tert.-Butanol, iso-Buten, iso-Butan oder iso-Butyraldehyd) zu olefinisch ungesättigten Aldehyden und/oder Carbonsäuren sowie den entsprechenden Nitrilen (Ammonoxidation, vor allem von Propen zu Acrylnitril und von iso-Buten bzw. tert.-Butanol zu Methacrylnitril). Besonders hervorgehoben sei die Herstellung von Acrylsäure, Acrolein und Methacrolein, sowie die Oxidation von n-Butan zu Maleinsäureanhydrid und diejenige von Butadien zu Furan. Sie eignen sich aber auch zur oxidativen Dehydrierung organischer Verbindungen.

Umsatz, Selektivität und Verweilzeit sind in dieser Schrift, falls nichts anderes erwähnt wird, wie folgt definiert:

$$\text{Umsatz U an Methacrolein (\%)} = \frac{\text{Molzahl umgesetztes Methacrolein}}{\text{Molzahl eingesetztes Methacrolein}} \times 100$$

$$\text{Selektivität S der Methacrylsäurebildung (\%)} = \frac{\text{Molzahl Methacrolein umgesetzt zu Methacrylsäure}}{\text{Molzahl Methacrolein insegamt umgesetzt}} \times 100$$

$$\text{Verweilzeit (sec)} = \frac{\text{mit Katalysator gefülltes Leervolumen des Reactors (l)}}{\text{durchgesetzte Synthesegasmenge ((Nl/h)}} \times 3600$$

Beispiel

a) Herstellung von erfindungsgemäßen Multimetalloxidmassen M und Multimetalloxidmassen MV zum Vergleich (den Gehalt an Wasserstoff, Ammonium und Sauerstoff der resultierenden Massen legt das jeweilige Herstellungsverfahren fest; die Werte wurden nicht regelmäßig ermittelt und sind daher in den angegebenen Stöchiometrien nicht regelmäßig enthalten)

MV1: Example 5 der EP-A 180 997 wurde nachgearbeitet. Als Antimonquelle wurde dabei reiner feinteiliger Senarmontit eines zahlenmittleren Partikeldurchmessers von 0,05 $\mu$m eingesetzt. Als Katalysatorgeometrie wurden Hohlzylinder der Ausmaße 7 mm (Höhe) x 7 mm (Außendurchmesser) x 3 mm (Innendurchmesser) gewählt.
Dem resultierenden Katalysator liegt nachfolgende Stöchiometrie zugrunde:

$$P_2 \ Mo_{12} \ W_{0,2} \ Fe_{0,5} \ B_{0,2} \ Ni_{0,5} \ Cs_2 \ Sb_1$$

MV2: Beispiel 14 der DE-A 26 10 249 wurde nachgearbeitet (Katalysatorgeometrie: 7 mm x 7 mm x 3 mm Hohlzylinder).
Resultierende Katalysatorstöchiometrie:

$$P_2 \ Mo_{12} \ Cs_2 \ Rh_{0,1} \ Fe_1$$

M1: Ausgangsmasse 2: In 1000 g Wasser wurden zunächst 900 g Ammoniumheptamolybdat, danach 294,5 g Ammoniumdodekawolframat und dann 165,6 g Cäsiumnitrat eingeführt. Die Temperatur der resultierenden wäßrigen Mischung wurde auf 33 bis 40°C eingestellt. In diese Mischung wurden 121,6 g 76 gew.-%ige Phosphorsäure und 5,8 g Borsäure eingerührt und die Temperatur der resultierenden Mischung auf 37 bis 45°C eingestellt. Anschließend wurden 68,7 g feinteiliges Antimontrioxid (reiner Senarmontit) mit einem zahlenmittleren Partikeldurchmesser von 0,05 $\mu$m zugesetzt, die resultierende Mischung auf 95°C erwärmt und während 1 h bei dieser Temperatur gehalten. Anschließend wurde die wäßrige Mischung bei einer Austrittstemperatur von 130°C sprühgetrocknet.
Der resultierenden Ausgangsmasse 2 liegt folgende Stöchiometrie zugrunde:

$$P_{2,22} \ Mo_{12} \ W_{0,22} \ B_{0,22} \ Cs_{2,22} \ Sb_{1,11}$$

Ausgangsmasse 1: 500 g Wasser wurden auf 60°C erwärmt und anschließend nacheinander 100 g Ammoniumheptamolybdat und eine wäßrige Lösung von 56,5 g Eisen(III)nitrat in 114,5 g einer 12,1 gew.-%igen wäßrigen Nickel(II)nitratlösung zugegeben und das resultierende Gemisch 2 h gerührt. Anschließend wurde das wäßrige Gemisch bei einer Austrittstemperatur von 140°C sprühgetrocknet und das anfallende Sprühpulver bei 460°C 6 h lang unter Luft calciniert.
Der resultierenden Ausgangsmasse 1 liegt folgende Stöchiometrie zugrunde:

$$Mo_{12} \ Fe_5 \ Ni_5$$

Die Ausgangsmasse 2 und die Ausgangsmasse 1 wurden im molaren Verhältnis 0,9 : 0,1 trocken gemischt. Nach Zusatz von 3 Gew.-% Graphit wurde die Trockenmasse zu Hohlzylindern (7 mm x 7 mm x 3 mm) tablettiert und 10 h bei 400°C im Luftstrom calciniert. Die Stöchiometrie der resultierenden Masse M1 entspricht derjenigen von MV1.

M2: Ausgangsmasse 2: Ausgehend von Ammoniumheptamolybdat, Cäsiumnitrat und 76 gew.-%iger Phosphorsäure wurde wie M1 ein Sprühpulver erzeugt, dem folgende Stöchiometrie zugrunde liegt:

$$P_{2,29} \ Mo_{12} \ Cs_{2,28}$$

Ausgangsmasse 1: 600 g Wasser wurden auf 60°C erwärmt und anschließend nacheinander 125 g Ammoniumheptamolybdat und eine Lösung von 113,07 g Eisen(III)nitrat in 9,88 g Rhodiumtrichlorid in 100 g Wasser zugegeben und das resultierende Gemisch 2 h gerührt. Anschließend wurde das wäßrige Gemisch bei einer Austrittstemperatur von 140°C sprühgetrocknet und das anfallende Sprühpulver bei 460°C 6 h lang unter Luft calciniert.
Der resultierenden Ausgangsmasse 1 liegt folgende Stöchiometrie zugrunde:

$$Mo_{12} \ Fe_8 \ Rh_{0,8}$$

Die Ausgangsmasse 2 und die Ausgangsmasse 1 wurden im molaren Verhältnis 0,875/0,125 trocken gemischt. Nach Zusatz von 3 Gew.-% Graphit wurde die Trockenmasse zu Hohlzylindern (7 mm x 7 mm x 3 mm) tablettiert und 10 h bei 400°C im Luftstrom calciniert.
Die Stöchiometrie der resultierenden Masse M2 entspricht derjenigen von MV2.

M3: Ausgangsmasse 2: In 1200 g Wasser wurden nacheinander 1000 g Ammoniumheptamolybdat, danach 55,21 g Ammoniummetavanadat und dann 128,8 g Cäsiumnitrat eingerührt. Die Temperatur der resultierenden wäßrigen Mischung wurden auf 40 bis 45°C eingestellt. In diese Mischung wurden 91,29 g 76 gew.-%ige Phosphorsäure und danach 2,49 g Ammoniumsulfat eingerührt und die Temperatur der resultierenden Mischung auf 45 bis 48°C eingestellt. Anschließend wurden 51,6 g feinteiliges Antimontrioxid (reiner Senarmontit) mit einem zahlenmittleren Partikeldurchmesser von 2,4 $\mu$m zugesetzt, die resultierende Mischung auf 95°C erwärmt und während 1 h bei dieser Temperatur gehalten. Anschließend wurde die wäßrige Mischung bei einer Austrittstemperatur von 110°C sprühgetrocknet.

Der resultierenden Ausgangsmasse 2 liegt folgende Stöchiometrie zugrunde:

$$P_{1,5} Mo_{12} V_1 Cs_{1,4} S_{0,04} Sb_{0,75}$$

Ausgangsmasse 1: Unter Rühren wurden in 800 g Wasser, die auf 50°C gehalten wurden, 166,67 g Ammoniumheptamolybdat gelöst und anschließend innerhalb von 30 min eine Lösung von 56,53 g Eisen(III)nitrat in 210,95 g einer 12,1 gew.-%igen wäßrigen Cobaltnitratlösung kontinuierlich zugegeben und das resultierende Gemisch 3 h gerührt. Anschließend wurde das wäßrige Gemisch bei einer Austrittstemperatur von 140°C sprühgetrocknet. Das resultierende Sprühpulver wurde nicht calciniert. Es liegt ihm folgende Stöchiometrie zugrunde:

$$Mo_{12} Fe_3 Co_{5,5}$$

Die Ausgangsmasse 2 und die Ausgangsmasse 1 wurden im molaren Verhältnis 1/0,17 trocken gemischt. Nach Zusatz von 3 Gew.-% Graphit wurde die Trockenmassen zu Hohlzylindern (5 mm x 5 mm x 3 mm) tablettiert und 6 h bei 380°C im Luftstrom calciniert.

M4: Ausgangsmasse 2: Wie bei M3.

Ausgangsmasse 1: Wie bei M3, jedoch wurde das anfallende Sprühpulver bei 460°C 6 h lang unter Luft calciniert.

Die Ausgansmasse 2 und die Ausgangsmasse 1 wurden im molaren Verhältnis 1/0,17 gemischt und wie bei M3 zu hohlzylinderförmigen Katalysatoren verarbeitet.

M5: Wie M4, die Ausgangsmasse 2 und die Ausgangsmasse 1 wurden jedoch im Verhältnis 1/1,5 vermischt.

M6: Ausgangsmasse 2: Wie bei M4.

Ausgangsmasse 1: Wie bei M4, mit dem Unterschied, daß noch eine entsprechende Menge Cs in Form von Cäsiumnitrat eingearbeitet wurde. Der resultierenden Ausgangsmasse 1 liegt die folgende Stächiometrie zugrunde:

$$Mo_{12} Fe_3 Co_{5,5} Cs_1$$

Die Ausgangsmasse 2 und die Ausgangsmasse 1 wurden im molaren Verhältnis 1/0,33 gemischt und wie bei M3 zu hohlzylinderförmigen Katalysatoren verarbeitet.

M7: Ausgangsmasse 2: In 1200 g Wasser wurden zunächst 1000 g Ammoniumheptamolybdat, danach 55,21 g Ammoniummetavanadat und dann 60 % einer Lösung von 110,24 g Cäsiumnitrat in 250 g Wasser eingerührt. Die Temperatur der resultierenden wäßrigen Mischung wurde auf 37 bis 42°C eingestellt. In diese Mischung wurden 91,29 g 76 gew.-%ige Phosphorsäure und danach 2,49 g Ammoniumsulfat eingerührt und die Temperatur der resultierenden Mischung auf 42 bis 45°C eingestellt. Anschließend wurden 96,31 g feinteiliges Antimontrioxid (25 % Valentinit und 75 % Senarmontit) mit einem zahlenmittleren Partikeldurchmesser von 3,2 $\mu$m zugesetzt und die Mischung auf 95°C erwärmt. Während der Aufheizphase wurden die verbliebenen 40 % der wäßrigen Cäsiumnitratlösung, aufgeteilt in drei gleich große Portionen, beim Erreichen von 80, 90 und 95°C jeweils auf einmal zugegeben. Anschließend wurde die wäßrige Mischung bei einer Austrittstemperatur von 120°C sprühgetrocknet.

Der resultierenden Ausgangsmasse 2 liegt folgende Stöchiometrie zugrunde:

$$P_{1,5} Mo_{12} V_1 Cs_{1,2} S_{0,04} Sb_{1,4}$$

Ausgangsmasse 1: Wie bei M4.

Die Ausgangsmasse 2 und die Ausgangsmasse 1 wurden im molaren Verhältnis 1/1,5 trocken gemischt. Nach Zusatz von 3 Gew.-% Graphit wurde die Trockenmasse zu Hohlzylindern (7 mm x 5 mm x 3 mm) tablettiert und 5 h bei 380°C im Luftstrom calciniert.

M8: Ausgangsmasse 2: Die Herstellung erfolgte wie bei M7, es wurde jedoch etwas weniger Antimontrioxid zugesetzt, so daß nachfolgende Stöchiometrie zugrunde liegt:

$$P_{1,5} Mo_{12} V_1 Cs_{1,4} S_{0,04} Sb_{0,75}$$

Ausgangsmasse 1: Wie bei M4, es wurde jedoch kein Eisen(III)nitrat mitverwendet und der Kobaltanteil wurde erhöht, so daß nachfolgende Stöchiometrie zugrunde liegt:

$$Mo_{12} Co_{12}$$

Die Ausgangsmasse 2 und die Ausgangsmasse 1 wurden im molaren Verhältnis 1/0,17 trocken gemischt und wie bei M4 zu hohlzylinderförmigen Katalysatoren verarbeitet.

M9: Ausgangsmasse 2: Wie bei M8.

Ausgangsmasse 1: Wie bei M8, es wurde jedoch anstelle von Kobaltnitrat Europium(III)nitrat eingesetzt und der Anteil des Eisens erhöht, so daß nachfolgende Stöchiometrie zugrunde liegt:

$$Mo_{12} Fe_7 Eu_1$$

Die Ausgangsmasse 2 und die Ausgangsmasse 1 wurden im molaren Verhältnis 1/0,33 trocken gemischt und wie bei M4 zu hohlzylinderförmigen Katalysatoren verarbeitet.

M10: Ausgangsmasse 2: In 1000 g Wasser wurden 1000 g Molybdäntrioxial und 52,65 g Vanadiumpentoxid suspendiert. Zu der wäßrigen Suspension wurden 112 g 76 gew.-%ige Phosphorsäure und 11,35 g 100 gew-%ige Schwefelsäure zugegeben. Anschließend wurde die Suspension bei 95°C gerührt und dabei nicht in Lösung gegangen geringfügigen Anteilen abfiltriert. Die wäßrige Lösung wurde auf 50°C abgekühlt und während 2 h kontinuierich 154,2 g Tetrapropylammoniumbromid zugegeben. Danach wurde 4 h bei 50°C gerührt. Anschließend wurde das wäßrige Gemisch bei einer Austrittstemperatur von 110°C sprühgetrocknet und 10 h bei 390°C calciniert. Der resultierenden Ausgangsmasse 2 liegt folgende Stöchiometrie zugrunde:

$$P_{1,5} Mo_{12} V_1 S_{0,2}$$

Ausgangsmassen 1: Wie bei M4.

Die Ausgangsmasse 2 und die Ausgangsmasse 1 wurden im molaren Verhältnis 1/0,17 trocken gemischt. Nach Zusatz von 3 Gew.-% Graphit wurde die Trockenmassen zu Hohlzylindern (7 mm x 5 mm x 3 mm) tablettiert und 5 h bei 380°C im Luftstrom calciniert.

M11: Ausgangsmassen 2: Wie bei M10, ein Teil des Phosphors wurde jedoch durch Arsen (Arsen(III)oxid) ersetzt und es wurde keine Schwefelsäure mitverwendet. Der resultierenden Ausgangsmasse 2 liegt folgende Stöchiometrie zugrunde:

$$P_{0,5} As_{0,5} Mo_{12} V_1 H_4$$

Ausgangsmasse 1: Wie bei M4.

Die Ausgangsmasse 2 und die Ausgangsmasse 1 wurden wie bei M4 zu hohlzylinderförmigen Katalysatoren verarbeitet.

b) Verwendung der Multimetalloxidmassen aus a) als Katalysatoren für die Gasphasenoxidation von Methacrolein zu Methacrylsäure

Die Katalysatoren wurden in einen Rohrreaktor gefüllt (10 mm Innendurchmesser, 100 g Katalysatorschüttung, Salzbadtemperierung) und bei Reaktionstemperaturen im Bereich von 280 bis 340°C unter Anwendung einer Verweilzeit von 3,6 Sekunden mit einem gasförmigen Gemisch der Zusammensetzung

5 Vol-% Methacrolein,
10 Vol.-% Sauerstoff,
30 Vol-%, Wasserdampf und
55 Vol-% Stickstoff

beschickt. Die Salzbadtemperatur wurde im wesentlichen in allen Fällen so eingestellt, daß ein einheitlicher Methacroleinumsatz von ca. 80,5 % resultierte. Eine geringere Salzbadtemperatur weist eine erhöhte Katalysatoraktivität aus. Das aus dem Rohrreaktor strömende Produktgasgemisch wurde gaschromatographisch analysiert. Die Ergebnisse für die Selektivität der Methacrylsäurebildung in Anwendung der verschiedenen Katalysatoren zeigt die nachfolgende Tabelle.

Tabelle

| Katalysator | Reaktionstemperatur (°C) | U (%) | S (%) |
|---|---|---|---|
| MV1 | 340 | 80,1 | 84,9 |
| MV2 | 310 | 84,1 | 85,6 |
| M1 | 305 | 80,1 | 86,3 |
| M2 | 302 | 84,1 | 87,9 |
| M3 | 298 | 81 | 88,8 |
| M4 | 302 | 80,5 | 88,3 |
| M5 | 308 | 81,3 | 87 |
| M6 | 324 | 81,8 | 82,7 |
| M7 | 314 | 80,9 | 87,5 |
| M8 | 322 | 81,2 | 87 |

Tabelle (fortgesetzt)

| Katalysator | Reaktionstemperatur (°C) | U (%) | S (%) |
|---|---|---|---|
| M9 | 296 | 81,1 | 88,3 |
| M10 | 283 | 89,8 | 85,4 |
| M11 | 285 | 90 | 86,9 |

Abschließend sei festgehalten, daß die Röntgenbeugungsaufnahme aller Multimetalloxidmassen M1 bis M11 sowohl den Fingerabdruck des Ammoniumsalzes der Molybdatophosphorsäure $((NH_4)_3PO_4(MoO_3)_{12} \cdot 4H_2O)$ als auch einen Defekt-Scheelit Fingerabdruck aufweisen.

**Patentansprüche**

1. Multimetalloxidmassen der allgemeinen Formel I

$$[A]_p [B]_q \qquad\qquad (I),$$

in der die Variablen folgende Bedeutung haben:

A $\quad Mo_{12} X_a^1 X_b^2 X_c^3 X_d^4 S_e X_f^5 O_x$

B $\quad X_{12}^6 X_g^7 X_h^8 O_y$

$X^1$ Phosphor, Arsen, Bor, Germanium und/oder Silicium,

$X^2$ Vanadium, Niob und/oder Wolfram,

$X^3$ Wasserstoff, von dem bis zu 97 mol-% ersetzt sein können durch Kalium, Rubidium, Cäsium und/oder Ammonium $(NH_4)$,

$X^4$ Antimon und/oder Wismut,

$X^5$ Rhenium und/oder Rhodium,

$X^6$ Molybdän, Wolfram, Niob und/oder Tantal,

$X^7$ Eisen, Kobalt, Nickel, Rhodium, Ruthenium, Magnesium, Calcium, Strontium, Barium, Zink, Cadmium, Quecksilber, Yttrium, Scandium und die seltenen Erdmetalle, wobei unter letzteren Cer, Lanthan und Europium bevorzugt werden,

$X^8$ Lithium, Natrium, Kalium, Rubidium, Cäsium und/oder Ammonium $(NH_4)$,

a 1 bis 6,

b 0 bis 6,

c 3 bis 5,

d 0 bis 6,

e 0 bis 3,

f 0 bis 3,

g 0 bis 20,

h 0 bis 4,

x,y Zahlen, die durch die Wertigkeit und Häufigkeit der von Sauerstoff verschiedenen Elemente in I bestimmt werden und

p,q von Null verschiedene Zahlen, deren Verhältnis p/q 12:0,1 bis 12:48 beträgt,

die den Anteil $[A]_p$ in Form dreidimensional ausgedehnter, von ihrer lokalen Umgebung aufgrund ihrer von ihrer lokalen Umgebung verschiedenen chemischen Zusammensetzung abgegrenzter, Bereiche A der chemischen Zusammensetzung

A $\quad Mo_{12} X_a^1 X_b^2 X_c^3 X_d^4 S_e X_f^5 O_x$

und den Anteil $[B]_q$ in Form dreidimensional ausgedehnter, von ihrer lokalen Umgebung aufgrund ihrer von ihrer lokalen Umgebung verschiedenen chemischen Zusammensetzung abgegrenzter, Bereiche B der chemischen Zusammensetzung

B $\quad X_{12}^6 X_g^7 X_h^8 O_y$

enthalten, wobei die Bereiche A, B relativ zueinander wie in einem Gemisch aus feinteiligem A und feinteiligem B verteilt sind.

2. Multimetalloxidmassen nach Anspruch 1, in denen wenigstens einer der beiden Anteile $[A]_p$, $[B]_q$, in Form dreidimensional ausgedehnter Bereiche enthalten ist, deren Größtdurchmesser 1 bis 200 $\mu$m beträgt.

3. Multimetalloxidmassen nach Anspruch 1 oder 2, in denen e 0,01 bis 1 beträgt.

4. Multimetalloxidmassen nach den Ansprüchen 1 bis 3, deren Röntgenbeugungsaufnahme den Fingerabdruck des Strukturtyps des Ammoniumsalzes der Molybdatophosphorsäure $((NH_4)_3PO_4(MoO_3)_{12} \cdot 4H_2O)$ aufweist.

5. Multimetalloxidmassen nach den Ansprüchen 1 bis 4, die das Element Antimon als Senarmontit enthalten.

6. Multimetalloxidmassen nach den Ansprüchen 1 bis 5, deren Röntgenbeugungsaufnahme einen Defekt-Scheelit-Fingerabdruck aufweist.

7. Verfahren zur Herstellung von Multimetalloxidmassen gemäß Anspruch 1, durch gekennzeichnet, daß man ein Oxometallat B

$$B \qquad X^6_{12} \ X^7_g \ X^8_h \ O_y$$

in feinteiliger Form vorbildet, und mit einem feinteiligen innigen Trockengemisch von Quellen der elementaren Konstituenten eines Oxometallates A

$$A \qquad Mo_{12} X^1_a \ X^2_b \ X^3_c \ X^4_d \ S_e \ X^5_f \ O_x$$

vermischt und das Gemisch bei einer Temperatur von 250 bis 450°C calciniert.

8. Verfahren zur gasphasenkatalytisch oxidativen Herstellung von Methacrylsäure aus Methacrolein, dadurch gekennzeichnet, daß als Katalysator ein Multimetalloxid gemäß der Ansprüche 1 bis 6 verwendet wird.

9. Verfahren nach Anspruch 8, dadurch gekennzeichnet, daß es sich bei dem verwendeten Katalysator um einen Vollkatalysator handelt, dessen Geometrie diejenige eines Hohlzylinders mit einem Außendurchmesser und einer Länge von 2 bis 10 mm und einer Wandstärke von 1 bis 3 mm ist.

**Claims**

1. A multimetal oxide composition of the formula I

$$[A]_p [B]_q \qquad\qquad (I),$$

where

A          is $Mo_{12} X^1_a \ X^2_b \ X^3_c \ X^4_d \ S_e \ X^5_f \ O_x$

B          is $X^6_{12} \ X^7_g \ X^8_h \ O_y$

$X^1$          is phosphorus, arsenic, boron, germanium and/or silicon,

$X^2$          is vanadium, niobium and/or tungsten,

$X^3$          is hydrogen, of which up to 97 mol% may have been replaced by potassium, rubidium, cesium and/or ammonium $(NH_4)$,

$X^4$          is antimony and/or bismuth,

$X^5$          is rhenium and/or rhodium,

$X^6$             is molybdenum, tungsten, niobium and/or tantalum,

$X^7$             is iron, cobalt, nickel, rhodium, ruthenium, magnesium, calcium, strontium, barium, zinc, cadmium, mercury, yttrium, scandium and/or a rare-earth metal, where, of the latter, cerium, lanthanum and europium are preferred,

$X^8$             is lithium, sodium, potassium, rubidium, cesium and/or ammonium ($NH_4$),

a             is from 1 to 6,

b             is from 0 to 6,

c             is from 3 to 5,

d             is from 0 to 6,

e             is from 0 to 3,

f             is from 0 to 3,

g             is from 0 to 20,

h             is from 0 to 4,

x and y      are numbers determined by the valency and frequency of the elements other than oxygen in I, and

p and q      are numbers other than zero whose ratio p/q is from 12:0.1 to 12:48,

which contains component $[A]_p$ in the form of three-dimensionally extended regions A of the chemical composition

A         $Mo_{12} X_a^1 X_b^2 X_c^3 X_d^4 S_e X_f^5 O_x$

which are delimited from their local environment due to their chemical composition which is different from their local environment, and component $[B]_q$ in the form of three-dimensionally extended regions B of the chemical composition

B         $X_{12}^6 X_g^7 X_h^8 O_y$

which are delimited from their local environment due to their chemical composition which is different from their local environment, where the regions A and B are distributed relative to one another as in a mixture of finely divided A and finely divided B.

2. A multimetal oxide composition as claimed in claim 1, where at least one of the two components $[A]_p$ and $[B]_q$ is in the form of three-dimensionally extended regions whose maximum diameter is from 1 to 200 μm.

3. A multimetal oxide composition as claimed in claim 1 or 2, where e is from 0.01 to 1.

4. A multimetal oxide composition as claimed in any of claims 1 to 3, whose X-ray diffraction pattern contains the finger-print of the molybdatophosphoric acid ammonium salt structural type $((NH_4)_3PO_4(MoO_3)_{12} \cdot 4H_2O)$.

5. A multimetal oxide composition as claimed in any of claims 1 to 4, where the antimony is in the form of senarmontite.

6. A multimetal oxide composition as claimed in any of claims 1 to 5, whose X-ray diffraction pattern contains a defect scheelite fingerprint.

7. A process for the preparation of a multimetal oxide composition as claimed in claim 1, which comprises pre-forming an oxometallate B

B $\quad X^6_{12}\ X^7_g\ X^8_h\ O_y$

in finely divided form, mixing this with a finely divided, intimate dry mix of sources of the elemental constituents of an oxometallate A

A $\quad Mo_{12}\ X^1_a\ X^2_b\ X^3_c\ X^4_d\ S_e\ X^5_f\ O_x,$

and calcining the mixture at from 250 to 450°C.

8. A process for the preparation of methacrylic acid from methacrolein by gas-phase catalytic oxidation, wherein the catalyst used is a multimetal oxide as claimed in any of claims 1 to 6.

9. A process as claimed in claim 8, wherein the catalyst used is an unsupported catalyst whose geometry is that of a hollow cylinder having an external diameter and a length of from 2 to 10 mm and a wall thickness of from 1 to 3 mm.

**Revendications**

1. Masses d'oxydes métalliques de formule I

$$[A]_p[B]_q\ (I) \tag{I}$$

dans laquelle les variables ont les significations suivantes:

| | |
|---|---|
| A | représente $Mo_{12}\ X^1_a\ X^2_b\ X^3_c\ X^4_d\ S_e\ X^5_f\ O_x,$ |
| B | représente $X^6_{12}\ X^7_g\ X^8_h\ O_y$ |
| $X^1$ | représente le phosphore, l'arsenic, le bore, le germanium et/ou le silicium, |
| $X^2$ | représente le vanadium, le niobium et/ou le tungstène, |
| $X^3$ | représente un atome d'hydrogène, dont jusqu'à 97 % en moles peuvent être remplacés par le potassium, le rubidium, le césium et/ou l'ammonium ($NH_4$), |
| $X^4$ | représente l'antimoine et/ou le bismuth, |
| $X^5$ | représente le rhénium et/ou le rhodium, |
| $X^6$ | représente le molybdène, le tungstène, le niobium et/ou le tantale, |
| $X^7$ | représente le fer, le cobalt, le nickel, le rhodium, le ruthénium, le magnésium, le calcium, le strontium, le baryum, le zinc, le cadmium, le mercure, l'yttrium, le scandium et les terres rares, parmi ces dernières, le cérium, le lanthane et l'europium étant préférés. |
| $X^8$ | représente le lithium, le sodium, le potassium, le rubidium, le césium et/ou l'ammonium ($NH_4$), |
| a | vaut de 1 à 6, |
| b | vaut de 0 à 6, |
| c | vaut de 3 à 5, |
| d | vaut de 0 à 6, |
| e | vaut de 0 à 3, |
| f | vaut de 0 à 3, |
| g | vaut de 0 à 20, |
| h | vaut de 0 à 4, |
| x, y | représentent des nombres qui sont déterminés par la valence et la fréquence des éléments autres que l'oxygène dans I, et |
| p, q | sont des nombres différents de zéro, dont le rapport p/q va de 12:0,1 à 12:48, |

qui contiennent la fraction $[A]_p$ sous forme de zones A à extension tridimensionnelle, délimitées de leur environnement local en raison de leur composition chimique différente de celle de leur environnement local, de composition chimique

A $\quad Mo_{12}\ X^1_a\ X^2_b\ X^3_c\ X^4_d\ Se\ X^5_f\ O_x,$

et la fraction $[B]_q$ sous forme de zones B à extension tridimensionnelle, délimitées de leur environnement local en raison de leur composition chimique différente de celle de leur environnement local, de composition chimique

B $\quad X^6_{12}\ X^7_g\ X^8_h\ O_y$

les zones A, B étant réparties les unes par rapport aux autres comme dans un mélange de A finement divisée et B finement divisée.

2. Masses d'oxydes multimétalliques selon la revendication 1, dans lesquelles est contenue au moins l'une des deux fractions $[A]_p$, $[B]_q$ sous forme de zones à extension tridimensionnelle, dont le diamètre maximum va de 1 à 200 µm.

3. Masses d'oxydes multimétalliques selon la revendication 1 ou 2, dans lesquelles e va de 0,01 à 1.

4. Masses d'oxydes multimétalliques selon les revendications 1 à 3, dont le diagramme de diffraction des rayons X présente les caractéristiques du type de structure du sel d'ammonium de l'acide molybdatophosphorique $[(NH_4)_3PO_4(MoO_3)_{12}.4H_2O]$.

5. Masses d'oxydes métalliques selon les revendications 1 à 4, qui contiennent l'élément antimoine sous forme de sénarmontite.

6. Masses d'oxydes métalliques selon les revendications 1 à 5, dont le diagramme de diffraction des rayons X présente les caractéristiques d'une scheelite défectueuse.

7. Procédé pour la préparation de masses d'oxydes métalliques selon la revendication 1, caractérisé en ce que l'on prépare au préalable un oxométallate B

$$B \qquad X_{12}^6 \ X_g^7 \ X_h^8 \ O_y$$

sous forme finement divisée, et on le mélange avec un mélange intime sec finement divisé de sources des constituants élémentaires d'un oxométallate A

$$A \qquad Mo_{12} \ X_a^1 \ X_b^2 \ X_c^3 \ X_d^4 \ Se \ X_f^5 \ O_x,$$

et on calcine le mélange à une température de 250 à 450°C.

8. Procédé pour la préparation par oxydation catalytique en phase gazeuse de l'acide méthacrylique à partir de la méthacroléine, caractérisé en ce que l'on utilise comme catalyseur un oxyde multimétallique selon les revendications 1 à 6.

9. Procédé selon la revendication 8, caractérisé en ce que le catalyseur utilisé consiste en un catalyseur massif dont la géométrie est celle d'un cylindre creux ayant un diamètre externe et une longueur de 2 à 10 mm et une épaisseur de paroi de 1 à 3 mm.